# EUROPEAN PATENT APPLICATION

(11) **EP 0 882 798 A1**
(43) Date of publication of application: **09.12.1998**
(21) Application number: 97201727.1
(22) Date of filing: 06.06.1997
(51) Int. Cl.: C12P 19/44, C12N 9/16, C11D 1/66, C12N 11/00

(54) **Process for the preparation of a monoester-containing esterification product of a polyoxyalkylated sucrose**

(71) Applicant: Akzo Nobel N.V., 6824 BM Arnhem (NL)
(72) Inventor: Boltersdorf, Dagmar, 52372 Kreuzau (DE); Wirtz, Monika, 52353 Düren (DE)
(74) Representative: Schalkwijk, Pieter Cornelis

(57) **Abstract**

Disclosed is a process for the preparation of a monoester-containing esterification product of a polyoxyalkylated sucrose in which the disaccharide is first alkoxylated and then esterified with a monocarboxylic acid having 6 to 24 carbon atoms or transesterified with a lower alkyl ester or glycerol ester thereof, wherein the esterification or transesterification process is carried out in the presence of a lipase. The process may be carried out in bulk or in a solvent, e.g., tert. butyl alcohol. Also claimed is the use of a monoester-containing esterification product of a polyoxyalkylated sucrose prepared by the above process as surfactant in a detergent composition.

## Description

The invention relates to a process for the preparation of a monoester-containing esterification product of a polyoxyalkylated sucrose in which the disaccharide is first alkoxylated and then esterified with a monocarboxylic acid having 6 to 24 carbon atoms or transesterified with a lower alkyl ester or glycerol ester thereof.

Such a process has earlier been proposed in US-A-2,908,681. According to the process disclosed in said document, first sucrose is converted with propylene oxide to form octakis-(2-hydroxypropyl)-sucrose, followed by acylation with an equimolar quantity of a fatty acid, acid anhydride, acid halide or an ester of the acid and a volatile alcohol. For the preparation of the monostearate ester in Example I use is made of equimolar quantities of methyl stearate and octakis-(2-hydroxypropyl)-sucrose in the presence of 0,5 wt.% of sodium methoxide, based on the total weight of reactants. At a temperature in the range of 100 to 120°C and a pressure of 80 mm the reaction was continued until the theoretical amount of methanol had been removed. The result was a thick yellow to brown syrup, which was bleached by the addition of 1 percent of 30 percent H₂O₂ before being cooled.

Japanese patent publication JP-A-1975-28413 likewise discloses a process in which sucrose is alkoxylated and then esterified with a monocarboxylic acid having 8 to 22 carbon atoms. The described alkoxylation process is carried out in the presence of a di-, tri- or polyol as solvent for the sucrose used as disaccharide. It is followed by esterification at a temperature in the range of 160 to 200°C or transesterification at a temperature in the range of 80 to 180°C. The high temperatures employed in the examples are inevitably attended with partial decomposition, and hence discolouring, of the disaccharide molecule. A further drawback to the process described in this publication is the high percentage of poorly separable side products, this on account of the large quantity of solvent which also forms part of the alkoxylating and esterifying process. The described preparative process is unsuitable for the preparation of an esterification product containing mainly monoester in which the percentage of monoester is virtually uncoloured and makes up at least 50 wt.% of the solids content of the overall reaction mixture. Especially for use as a surface-active material it is of the essence that no or very few di-and/or higher esters are formed, since the surface-active properties of such compounds are unsatisfactory.

The invention now provides a process of the known type mentioned in the opening paragraph by means of which a virtually uncoloured esterification product containing mainly monoester can be obtained within a comparatively short period of time.

The invention consists in that in a process of the known type mentioned in the opening paragraph the esterification or transesterification process is carried out in the presence of a lipase.

It should be noted that regioselective esterification of certain disaccharides in the presence of a catalytic quantity of a lipase is known in itself from an article by Woudenberg et al. in *Biotechnology and Bioengineering,* Vol. 49, pp. 328-333 (1996). For instance, maltose is acylated with ethyl dodecanoate to form 6'-monododecanoate at very high conversion. By contrast, the acylation of sucrose proceeds at such a low pace that only after 7 days a conversion of 37% is attained. In addition, it was found that besides the monoester large quantities of diester are formed. In consequence, such a process is unsuited to be used on a commercial scale.

In any case, it has to be deemed extremely surprising that using the process according to the present invention in which instead of sucrose use is made of a polyoxyalkylated derivative thereof will give a high conversion at a high selectivity within a comparatively short period of time.

The process according to the invention may be carried out solvent-free or in the presence of a solvent. If use is made of a solvent, preference is given to a solvent selected from the group of petroleum ether, n-hexane, n-heptane, toluene, isopropyl ether, methyl tert. butyl ether, methyl isobutyl ketone, dimethyl formamide, tetrahydrofuran, iso-octane, and tert. amyl alcohol. Optimum results were obtained with tert. butanol.

The preparation of polyoxyalkylated sucrose can be carried out not only as disclosed in the US patent specification discussed above, but also by using a process such as described in US-A-4,239,907 or US-A-4,332,936.
The former of these last two patent publications relates to a process in which sucrose is mixed, in the presence of ammonia or an alkanolamine and water, with ethylene oxide and, optionally, a second alkylene oxide at a temperature ≤ 110 °C to form the desired polyether polyol.
The process described in the latter patent publication involves sucrose in the presence of an amine catalyst being dissolved wholly or in part in a solvent containing 5 to 70 g of dimethyl formamide per 100 g of sucrose, followed by conversion with an alkylene oxide.

The starting product for esterification preferably is an alkoxylated sucrose having 1 to 50 alkoxyl groups per molecule of sucrose. The alkoxyl groups may have 2 to 4 carbon atoms per alkoxyl group. Optimum results are obtained when the alkoxyl groups are derived from cyclic ethers such as ethylene oxide and mixtures of ethylene oxide and propylene oxide. Generally speaking, optimum results are obtained with 8 to 32 alkoxyl groups present per disaccharide molecule. In that case, per OH group of the disaccharide there are 1 to 4 alkoxyl groups bonded via ether bonds.

The lipase which is suitable for use in the process according to the invention preferably is of the *Candida antarctica* type. The lipase is preferably used in the immobilised form, which is obtained by adsorption from an aqueous solution onto a powder or granulate of a porous aliphatic polyolefin pretreated with a polar, organic, water-miscible solvent in which the polymer is insoluble and which does not inactivate the lipase.
The porous aliphatic polyolefin preferably is polypropylene over 90 wt.% of the particles of which have a size in the range of 100 to 1000 µm.

The fatty acids can be used as such or in the esterified form with a lower alcohol having 1 to 4 carbon atoms or with glycerol. Examples of suitable acids are caproic acid, heptanoic acid, caprylic acid, nonylic acid, capric acid, undecylic acid, lauric acid, tridecylic acid, myristic acid, n-pentadecylic acid, palmitic acid, margaric acid, hydroxystearic acid, stearic acid, oleic acid, linoleic acid, tall oil, coconut oil, mixtures of fatty acids derived from vegetable, animal, and synthetic fats and oils.

The esterification of polyoxyalkylated sucrose may be performed in bulk or in a solvent. In a bulk process, the fatty acid or ester thereof is dissolved or dispersed in the alkoxylated sucrose and heated, under vigorous stirring, to a temperature in the range of 60 to 80°C, followed by the addition of the enzyme while maintaining the temperature in the range of 40 to 80°C and the application of a vacuum to remove the water formed. In a solvent process, the oxyalkylated product is dissolved and/or dispersed in, e.g., tert. butyl alcohol, followed by the addition of a quantity of a fatty acid or ester thereof ranging from equimolar to more than molar excess. To the resulting homogeneous mixture is then added the enzyme, after which the whole is maintained at a temperature in the range of 40 to 80°C with the water released on esterification being removed by applying of a vacuum or, optionally, being trapped, e.g., by a molecular sieve in a Soxhlet apparatus. The progress of the reaction can be monitored by determining the acid number. As soon as the reaction is drawing to an end, there is cooling to about 70°C, the catalyst is filtered off, and the solvent is removed by evaporation *in vacuo.*

Surprisingly, it has been found that the esteriflcation reaction can be performed substantially more rapidly when the quantity of tert. butyl alcohol is reduced to 10 to 50 ml per 100 g of substrate.

Further, it has been found that to obtain a small percentage of non-esterified alkoxylate in the reaction product it is preferred to employ a molar ratio of sucrose to acid of 1:1,5 and 1:2.
Preference is given in that case to esterification or transesterification in the presence of 25 to 35 ml of tert. butyl alcohol per 100 g of substrate.

The invention will be elucidated with reference to the following examples. Needless to say, these are intended to be exemplary and not as limitations upon the scope of the invention.

### Example I

### Preparation of octakis-(2-hydroxyethyl)sucrose

An ethylene oxide pilot plant reactor was filled with an aqueous solution heated to between 60 and 70°C and containing 146 moles of saccharose (refined sugar) dissolved in water until a solution was obtained containing 73 % solid matter. After the solution had been purged with nitrogen for 15 minutes at 70°C, there was added an aqueous solution containing 45% KOH, followed by 1169 moles of ethylene oxide, at a temperature in the range of 70 to 95°C over a period of 1 hour. Subsequently, the water was evaporated over a period of 2 hours at a temperature between 80 and 100°C and a pressure between 20 and 90 mm Hg. After cooling to 80°C the reaction product was decanted under a nitrogen pressure of 5 bar.

### Example II

A 500 ml three-neck flask equipped with a condenser, a thermometer, and a blade stirrer was filled with 0,2 mole (173,9 g) of sucrose etherified with 12 ethylene oxide groups (12 EO), prepared in a similar manner to the octakis-(2-hydroxyethyl)sucrose in Example I, and 0,2 mole (40,06 g) of lauric acid. The mixture was stirred and heated to 70°C. To the homogeneous mixture were added 4,01 g of *Candida antarctica* lipase (Novozym 435 ex Novo). The mixture was stirred and the water produced in the esterification reaction removed under reduced pressure (10 mbar). The progress of the reaction was monitored by determining the acid content. After a 3-hour reaction at 70°C and a pressure of 10 mbar a product having an acid value < 1 was obtained. The catalyst was filtered off at 70°C using a thermostated Seitz pressure filter funnel and filter layer T 5500. Obtained was a homogeneous, clear, colourless, viscous liquid containing 0,35 wt.% of unreacted lauric acid. A 1 wt.% clear aqueous solution was made by dissolving the product in water. The thus obtained solution possessed high foaming properties.

### Example III

A 1 l three-neck flask equipped with a reflux condenser, a Soxhlet apparatus fitted with an activated molecular sieve 4 A, a stirrer, and a thermometer was filled with 0,12 mole (83,32 g) of octakis-(2-hydroxyethyl)sucrose of Example I and 500 ml tert. butyl alcohol. The whole was heated to 70°C. To the homogeneous mixture were added 0,12 mole (24,04 g) of lauric acid and, after its dissolution, 2,40 g of *Candida antarctica* lipase (Novozym 435 ex Novo). The mixture was heated to a temperature of about 80°C, with the water released in the esterification process being trapped by the molecular sieve in the Soxhlet apparatus. The progress of the reaction was monitored by determining the acid content, in which process the molecular sieve had to be replaced as many as three times. After 13 hours a product having an acid value < 2 was obtained. After cooling to 70°C the catalyst was filtered off and the solvent was evaporated *in vacuo*. Almost 70 wt.% of the end product was made up of the monoester of octakis-(2-hydroxyethyl)sucrose, while 27 wt.% was made up of unconverted octakis-(2-hydroxyethyl)sucrose. The content of free acid was determined to be 0,7 wt.% (calculated on the solids content).

### Example IV

The esterification process was carried out in a manner analogous to the one disclosed in Example III, with the proviso that this time no use was made of a molecular sieve and the quantity of tert. butyl alcohol was reduced to 10 ml per 100 g of substrate. During the 5-hour esterification at 70°C the reaction mixture was not entirely homogeneous, but there was no phase separation. During the esterification the released water was not removed. After a vacuum (10 mbar) had been applied, the water and the tert. butyl alcohol were removed, whereupon the content of free acid was determined to be 1 wt.% (calculated on the solids content).

### Example V

The esterification process of Example IV was repeated, with the proviso that this time use was made of 20 ml tert. butyl alcohol per 100 g of substrate. The content of unconverted acid was determined to be 0,4 wt.% (calculated on the solids content).

### Example VI

The esterification process of Example IV was repeated, with the proviso that this time use was made of 30 ml tert. butyl alcohol per 100 g of substrate. The content of unconverted acid was determined to be 0,7 wt.% (calculated on the solids content). Example VII

The esterification process of Example IV was repeated, with the proviso that this time use was made of 40 ml tert. butyl alcohol per 100 g of substrate. The content of unconverted acid was determined to be 0,5 wt.% (calculated on the solids content).

### Example VIII

The esterification process of Example IV was repeated, with the proviso that this time use was made of 50 ml tert. butyl alcohol per 100 g of substrate. The content of unconverted acid was determined to be 0,5 wt.% (calculated on the solids content).

### Example IX

The esterification process of Example IV was repeated, with the proviso that this time use was made of an octakis-(2-hydroxyethyl)sucrose : lauric acid ratio of 1:1,5. The content of unconverted acid was determined to be 0,4 wt.% (calculated on the solids content).
The resulting product contained 17 wt.% of unconverted octakis-(2-hydroxyethyl)sucrose.

### Example X

The esterification process of Example IX was repeated, with the proviso that this time use was made of an octakis-(2-hydroxyethyl)sucrose : lauric acid ratio of 1:2. The content of unconverted acid was determined to be 1 wt.% (calculated on the solids content).
The resulting product contained 15 wt.% of unconverted octakis-(2-hydroxyethyl)sucrose.

## Claims

1. A process for the preparation of a monoester-containing esterification product of a polyoxyalkylated sucrose in which the disaccharide is first alkoxylated and then esterified with a monocarboxylic acid having 6 to 24 carbon atoms or transesterified with a lower alkyl ester or glycerol ester thereof, characterised in that the esterification or transesterification process is carried out in the presence of a lipase.

2. A process according to claim 1, characterised in that the process is carried out in the presence of a solvent.

3. A process according to claim 2, characterised in that the solvent is selected from the group of petroleum ether, n-hexane, n-heptane, toluene, isopropyl ether, methyl tert. butyl ether, methyl isobutyl ketone, dimethyl formamide, tetrahydrofuran, iso-octane, and tert. amyl alcohol.

4. A process according to claim 2, characterised in that the solvent is tert. butyl alcohol.

5. A process according to claim 1, characterised in that in the esterification or transesterification process use is made of alkoxylated sucrose containing 1 to 50 alkoxyl groups each having 2 to 4 carbon atoms per disaccharide molecule.

6. A process according to claim 1, characterised in that the alkoxy groups are derived from cyclic ethers such as ethylene oxide and mixtures of ethylene oxide and propylene oxide.

7. A process according to claim 1, characterised in that the lipase is *Candida antarctica.*

8. A process according to claim 7, characterised in that the lipase is immobilised on a carrier.

9. A process according to claim 1, characterised in that the esterification or transesterification process is carried out at a temperature in the range of 40 to 80 °C.

10. A process according to claim 4, characterised in that the esterification or transesterification process is carried out in the presence of 10 to 50 ml tert. butyl alcohol per 100 g of substrate.

11. A process according to claim 1, characterised in that in the esterification or transesterification process use is made of a molar ratio of disaccharide to acid of 1:1,5 and 1:2.

12. A process according to claim 4, characterised in that the esterification or transesterification process is carried out in the presence of 25 to 35 ml tert. butanol per 100 g of substrate.

13. Use of a monoester-containing esterification product of a polyoxyalkylated sucrose prepared by a process according to one or more of the preceding claims as a surfactant in a detergent composition.
